# EUROPEAN PATENT APPLICATION

(11) **EP 4 541 879 A1**
(43) Date of publication of application: **23.04.2025**
(21) Application number: 23823456.1
(22) Date of filing: 24.02.2023
(51) Int. Cl.: C12M 1/00, C12N 15/10, C12Q 1/02, C12Q 1/6806, C12Q 1/6869

(54) **CONTAINER FOR SINGLE CELL ANALYSIS AND SINGLE CELL ANALYSIS METHOD USING SAME**

(30) Priority: 16.06.2022 JP 2022097129
(71) Applicant: Hitachi, Ltd., Tokyo 100-8280 (JP)
(72) Inventor: TANIGUCHI Kiyomi, Tokyo 100-8280 (JP); SHIRAI Masataka, Tokyo 100-8280 (JP)
(74) Representative: Mewburn Ellis LLP
(86) International application number: PCT/JP2023/006649
(87) International publication number: WO 2023/243152

(57) **Abstract**

The present invention relates to a container for single cell analysis and a single cell analysis method using the same. The present invention also relates to an automated device for performing single cell analysis using the container for single cell analysis. Specifically, the present invention provides a container for single cell analysis which includes: a reaction substrate including a cell trapping portion on which a cell is trapped, and one or a plurality of microreaction chambers arranged immediately below the cell trapping portion and filled with a solid phase; and a cell holding portion having the reaction substrate as a bottom and configured to hold a solution containing cells.

## Description

### Technical Field

The present invention relates to a container for single cell analysis and a single cell analysis method using the same. The present invention also relates to an automated device for performing single cell analysis using the container for single cell analysis.

### Background Art

Single cell analysis is a technique for detecting and quantifying biomolecules in cells per single cell with high accuracy. Various techniques have been developed for single cell analysis. In particular, a device for performing single cell analysis of a large number of cells on one device at a time has been developed and commercialized (NPL 1). This technique is a technique based on microfluidics using semiconductor patterning, and can cope with various reactions by constructing a large number of microchannels, reaction chambers, and valves in a two-dimensional plane. Particularly, in order to prepare a sample for single cell analysis, a structure for trapping single cells in a flow path in a two-dimensional plane, and a channel in which a reaction chamber for disrupting (Lysis) cells, a reaction chamber for reverse transcription reaction, and a reaction chamber for PCR amplification are connected in series downstream of the structure are constructed. In a case where the reaction chambers are arranged in series in the channel in the plane to prepare a nucleic acid sample from cells for single cell analysis, the number of reaction chambers needs to be increased in accordance with the number of cells to be treated. Meanwhile, since there is a limit to reduce the size of the necessary reaction chamber from the size of the cell and the amount of the biological substance therein, the area of the device increases as the number of cells increases. At this time, since this technique uses semiconductor patterning, there is a problem that the cost is proportional to the area of the device, and the device cost increases as the number of cells increases.

To overcome this problem, a technique has been proposed in which a flow path is constructed not parallel to a surface of a device substrate but perpendicular to the surface, thereby increasing the degree of integration and reduce the device cost (PTLs 1 to 4). The device described in each of these literatures includes a cell trapping portion and a nucleic acid trapping portion. A single cell is trapped and isolated by the cell trapping portion, and mRNA is trapped by tagged DNA immobilized on beads or a surface of a porous material by the nucleic acid trapping portion arranged immediately below the cell trapping portion, whereby a different tag is inserted into each cell and each mRNA molecule. Further, reverse transcription is performed on the device, as a result of which tag and gene sequences are synthesized as a single cDNA strand. This cDNA is subjected to nucleic acid amplification, and the sequences are analyzed with a next generation sequencer. The number of molecules of each cDNA can be counted by counting the measured reads for each cell identification tag and molecular identification tag. Particularly, this method has high conversion efficiency from mRNA to cDNA, and can approximately count mRNAs with high accuracy.

### Citation List

### Patent Literature

PTL 1: WO 2014/020657 A
PTL 2: WO 2016/207986 A
PTL 3: WO 2014/141386 A
PTL 4: WO 2019/116800 A(JP 2019-103415 A)

### Non Patent Literature

NPL 1: D. Ramskold et al., Nat Biotechnol, Vol. 30, No. 8, p. 777 to 782, 2012

### Summary of Invention

### Technical Problem

Analysis of the cancer microenvironment has attracted attention in therapeutic research on cancer, particularly in clinical research on personalized medical methods. This cancer microenvironment is composed of cancer cells, normal cells, and various immune cells. In order to analyze the functions of these cells, single cell analysis has attracted attention as a means for analyzing the state of genes expressed in cells. Further, antigen-presenting cells, such as dendritic cells infiltrated in the cancer microenvironment, move to the lymph nodes, and a cancer antigen-selective immune response (proliferation of killer T-cells, production of antibodies, etc.) occurs. Here, the proliferated immune cells are induced by signal transducers such as chemokines and return to cancer tissues. Accordingly, it is also expected to grasp a change in the cancer microenvironment as a partial change in the state of immune cells in peripheral blood, which has started to attract attention.

So far, many single cell analysis devices have been configured on the premise that cells are trapped on a chip in a device, mRNA trapped on the surface of beads filled in a microreaction chamber (nucleic acid trapping portion) immediately below the chip is reverse-transcribed in the device to synthesize complementary cDNA, and the nucleic acid treatment reaction such as PCR is performed in the device (PTL 1 and PTL 2). Further, it is possible to perform various nucleic acid treatments by immersing the chip in the device in a solution in another tube (resin container) and suspending the beads in the solution (PTL 3 and PTL 4).

However, to execute the nucleic acid treatment reaction in the device, it is necessary to develop a device suitable for the reaction, and the degree of freedom of the nucleic acid treatment is low.

In addition, to collect nucleic acids trapped on a solid phase, such as beads and fibers in the device, in another tube, it takes time and effort to extract the chip filled with the solid phase (beads) in the device from the device using a tool such as tweezers, immerse the chip in the solution in the container, and further disperse the solid phase (beads) in the solution in the container.

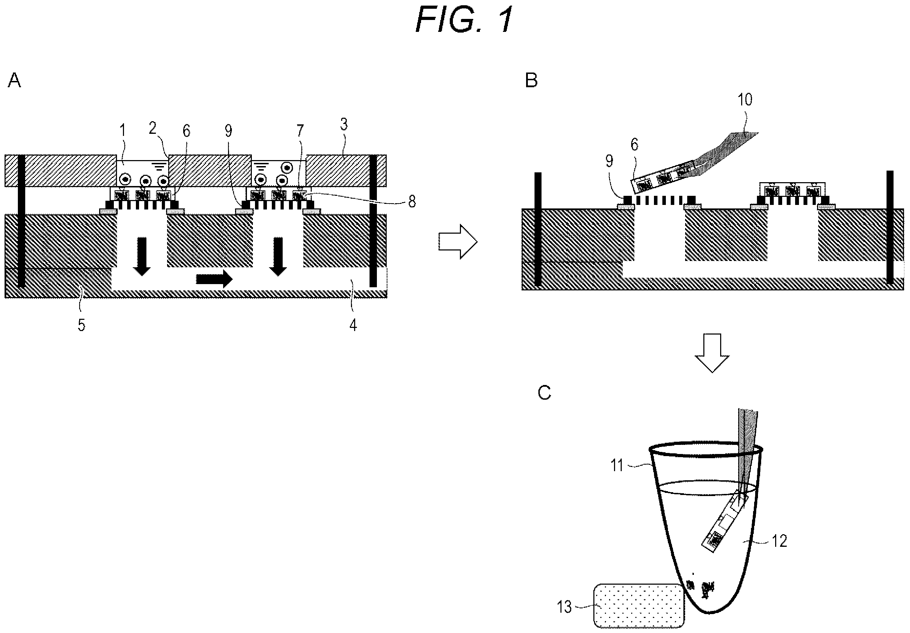

FIG. 1 illustrates a configuration of a single cell analysis device and how to use the device when beads are dispersed in a container different from the single cell analysis device in the related art. FIG. 1A is a cross-sectional view of the single cell analysis device. This device includes an upper plate 3 for forming a well (container) 2 for holding a cell suspension 1, a lower plate 5 for forming a solution aspiration flow path 4, and a single cell analysis chip 6 sandwiched between the upper and lower plates. The single cell analysis chip 6 includes a plurality of through-holes. The through-holes include: a cell trapping portion 7 provided at a portion in contact with the cell suspension; and a nucleic acid trapping portion 8 immediately below the cell trapping portion 7, the nucleic acid trapping portion 8 being filled with beads on which a DNA probe for trapping mRNA is immobilized. A porous membrane 9, which is located immediately below the single cell analysis chip 6 and in which a large number of pores smaller than the bead size are formed for holding the beads in the nucleic acid trapping portion, is brought into close contact with the single cell analysis chip 6. An aspiration pump is connected to the flow path 4 and a negative pressure is applied to the back surface of the single cell analysis chip 6, and thus the cell suspension 1 is aspirated through the cell trapping portion 7 and the nucleic acid trapping portion 8. At this time, cells are trapped one by one in the cell trapping portion 7 having a diameter smaller than the diameter of the cells. When the cells are trapped, the solution flow passing through the corresponding cell trapping portion stops. Thus, the remaining cells are aspirated by the cell trapping portion 7 that has not yet trapped any cell, and the cells are isolated. All the cells are aspirated while being continuously aspirated by the pump (dispensed such that the number of cells in the cell suspension is smaller than the number of the cell trapping portions). When a cell disruption (lysis) solution is added to the well, the cells are disrupted, and the cell disruption solution from each cell passes through the nucleic acid trapping portion. At this time, mRNA is trapped by a polyT sequence-containing DNA probe immobilized on beads. In the DNA probe immobilized on the beads, a sequence (cell identification tag) different for each position in the chip of the microreaction chamber (nucleic acid trapping portion) is inserted on the 3 'end side from the poly T sequence. Further, a solution for washing the lysis solution is added, and the washed lysis solution is discharged from the nucleic acid trapping portion. Thereafter, the pump is stopped, and the pressure is returned to atmospheric pressure. Then, a reverse transcriptase solution is added thereto. A syringe is connected to the flow path 4 such that the microreaction chamber (nucleic acid trapping portion) is filled with the reverse transcription solution, and a negative pressure is applied thereto for about several seconds. An opening in the upper portion of the well is sealed with a PCR seal or the like such that the reverse transcriptase is not evaporated. The entire device is kept at a temperature suitable for the reverse transcription reaction until the reverse transcription reaction is completed. After the completion of the reverse transcription reaction, the device temperature is returned to room temperature. The seal is removed, and the flow path 4 is connected to the pump again, whereby the reverse transcription reaction solution remaining in a well 2 is completely aspirated.

Next, as illustrated in FIGS. 1B and 1C, in order to pick up the chip, the fixed upper plate 3 is removed, and the chip 6 is gripped with tweezers 10 and immersed in a bead suspension and collection buffer 12 in a resin tube for bead collection 11. The enzyme solution is sufficiently aspirated for the beads in the chip before pick up of the chip, most of the beads filled in the nucleic acid trapping portion 8 of the chip are held in the microreaction chamber (nucleic acid trapping portion). To suspend the beads in a bead suspension buffer, vibration needs to be applied to the chip. In a situation where the chip is not fixed, the chip moves, and it is difficult to appropriately apply vibration. Further, in the case of using a chip made of high-elastic polydimethylsiloxane (PDMS) in consideration of the enzyme reaction in the nucleic acid trapping portion, the chip is flexible, and thus it is further difficult to apply vibration. Accordingly, the beads are diffused from the nucleic acid trapping portion into the solution by repeatedly bending and stretching the chip using tweezers, and the beads are collected by the magnet 13 utilizing the fact that the beads are magnetic beads. Due to the bending and stretching of the chip, not only the diffusion of the beads takes time, but also the tweezers used for extracting the chip need to be immersed in the bead suspension. Consequently, there is a problem that beads between the chips and substances in the solution are mixed.

### Solution to Problem

The present inventors have found that, in the conventional single cell analysis device, a bottom of a container for storing a cell suspension is used as a single cell analysis chip, and an aspiration port for aspirating a solution in the container through the chip from a bottom-surface back side of the container (back side of the chip) is brought into close contact with the chip in a removable manner, and thus the container is removed after a reaction on the chip and introduced into another container, vibration is applied to the chip, and a solid phase such as a bead can be easily collected in the other container.

Therefore, in one aspect, the present invention provides a container for single cell analysis which includes: a reaction substrate including a cell trapping portion on which a cell is trapped, and one or a plurality of microreaction chambers arranged immediately below the cell trapping portion and filled with a solid phase; and a cell holding portion having the reaction substrate as a bottom and configured to hold a solution containing cells.

**In** another aspect, the present invention provides a single cell analysis method using a single cell analysis device, in the single cell analysis device to which the container for single cell analysis according to the present invention is fixed, the method including the steps of:
introducing a solution containing cells into the container;
trapping the cells in the cell trapping portion;
reacting nucleic acid derived from the trapped cells in the microreaction chamber;
removing the container from the single cell analysis device after the reaction; and
collecting the solid phase from the removed container.

**In** still another aspect, the present invention provides an automated single cell analysis device including:
a single cell analysis device configured to fix the container for single cell analysis according to the present invention;
a mechanism that is configured to remove the container from the single cell analysis device and move the container to another container;
a mechanism that is configured to vibrate the container in the other container; and
a control device,
in which the control device is configured to remove the container from the single cell analysis device after a reaction in the single cell analysis device, and to control a movement of the container to the other container and a vibration of the container in the other container.

This specification encompasses the disclosure of Japanese Patent Application No. 2022-097129 filed on June 16, 2022, based on which the present application claims priority.

### Advantageous Effects of Invention

The present invention provides a container for single cell analysis for use in a single cell analysis device, and a single cell analysis method, and an automated single cell analysis device using the same. According to the present invention, the solid phase in the single cell analysis device can be easily and quickly collected in another container, the time of the nucleic acid treatment reaction is shortened, and various nucleic acid treatment reactions can be applied. Therefore, the present invention is useful in the fields of single cell analysis, particularly gene expression analysis, cell function analysis, biological tissue analysis, diagnosis of diseases, drug discovery, and the like.

### Brief Description of Drawings

[FIG. 1] FIG. 1 illustrates a configuration of a single cell analysis device and how to use the device when beads are dispersed in a container different from the single cell analysis device in the related art.
[FIG. 2] FIG. 2 illustrates a specific structural example of a container for single cell analysis according to the present invention and how to use the container.
[FIG. 3] FIG. 3 illustrates a structural view from above when a container for single cell analysis 21 is lifted from the single cell analysis device.
[FIG. 4] FIG. 4 is a view of the container for single cell analysis 21 of Example 1 as viewed from above.
[FIG. 5] FIG. 5 is a cross-sectional view of the container for single cell analysis 21 of Example 1 in a state in which no cell suspension is contained.
[FIG. 6] FIG. 6 is a cross-sectional view of a single cell analysis device of Example 1.
[FIG. 7] FIG. 7 is a graph showing results of performing gene expression analysis derived from single cells using a container for single cell analysis of Example 1.
[FIG. 8] FIG. 8 is a flowchart showing a flow example in an automated device for single cell analysis using a container for single cell analysis.
[FIG. 9] FIG. 9 is a view illustrating a configuration example of an automated device for single cell analysis using a container for single cell analysis.
[FIG. 10] FIG. 10 shows a top view (A) and a cross-sectional view (B) of connected containers 201, i.e. an example of a container for single cell analysis, as viewed from an upper surface of a chip 30.
[FIG. 11] FIG. 11 is a view illustrating another configuration example of the automated device for single cell analysis using the container for single cell analysis.

### Description of Embodiments

In one aspect, the present invention provides a container for single cell analysis which includes: a reaction substrate including a cell trapping portion on which a cell is trapped, and one or a plurality of microreaction chambers arranged immediately below the cell trapping portion and filled with a solid phase; and a cell holding portion having the reaction substrate as a bottom and configured to hold a solution containing cells.

The reaction substrate including the cell trapping portion and the reaction substrate can be a reaction substrate used in a single cell analysis device known in the art. Such a reaction substrate is described in, for example, WO 2014/020657 A (PTL 1), WO 2014/141386 A (PTL 3), and WO 2019/116800 A (PTL 4). In one embodiment, the reaction substrate may be made of resin, particularly polydimethylsiloxane (PDMS), cycloolefin, polypropylene, polycarbonate, or polyethylene. Further, the size of the microreaction chamber (nucleic acid trapping portion) filled with the solid phase (beads or the like) on the reaction substrate may have, for example, a diameter of 0.5 mm or less, a depth of 0.05 mm or more, and an aspect ratio of 0.1 or more. The solid phase to be filled is not limited. For example, beads (preferably magnetic beads), fibers (mesh, sponge, hollow fiber, etc.), or the like can be used as the solid phase. When beads are used as the solid phase to be filled, the size of the beads may be preferably, for example, 3 µ or less. The cell trapping portion preferably has a shape and a size such that a single cell is trapped per cell trapping portion.

The reaction substrate may include a layer having an aspiration through-hole provided on a side opposite to the cell holding portion. Such a configuration is described in the literatures as described above and is known to those skilled in the art. A solution portion of a solution containing cells, a reagent introduced onto the reaction substrate, a wash solution, and the like may be aspirated from the through-hole, and thus the reaction on the reaction substrate can be quickly performed with high accuracy.

The cell holding portion may be configured to hold the solution containing cells with the reaction substrate as a bottom. The shape of the cell holding portion may not be particularly limited as long as it is compatible with the single cell analysis device to which the cell holding portion is fixed and can hold a solution. The shape may be any shape, such as a cylinder, a cone, a quadrangular prism, or a triangular prism. The size of the cell holding portion can be appropriately set in accordance with the sizes of the reaction substrate and the single cell analysis device, the amount of solution to be introduced, and the like. The inner wall of the cell holding portion may be subjected to a water repellent treatment such that cells, a solution, a reaction reagent to be introduced onto the reaction substrate, and the like are hardly attached.

The container for single cell analysis according to the present invention may be removably fixed to the single cell analysis device, and can be removed after reaction. Accordingly, in one embodiment, the container for single cell analysis may further include a fixture for fixing to the single cell analysis device. **In** particular, the fixture may preferably be a fixture that can stably fix the device without moving during a reaction on the reaction substrate or a washing operation before and after the reaction. Such a fixture is not particularly limited, and may be a screw, a projection, a magnet, a removable adhesive, or the like. Further, in one embodiment, the container for single cell analysis may further include an auxiliary tool for removing from a single cell analysis device. Particularly, in the automated device, it may be preferable to use an auxiliary tool capable of easily and quickly removing the container for single cell analysis from the device. Such an auxiliary tool is not particularly limited, and may be a projection, a magnet, or the like. Even in the absence of the auxiliary tool, the container can be removed from the single cell analysis device by utilizing a mechanism to grasp the container from the outside or a mechanism to hold the container by aspiration. Thus, the auxiliary tool is not necessarily used.

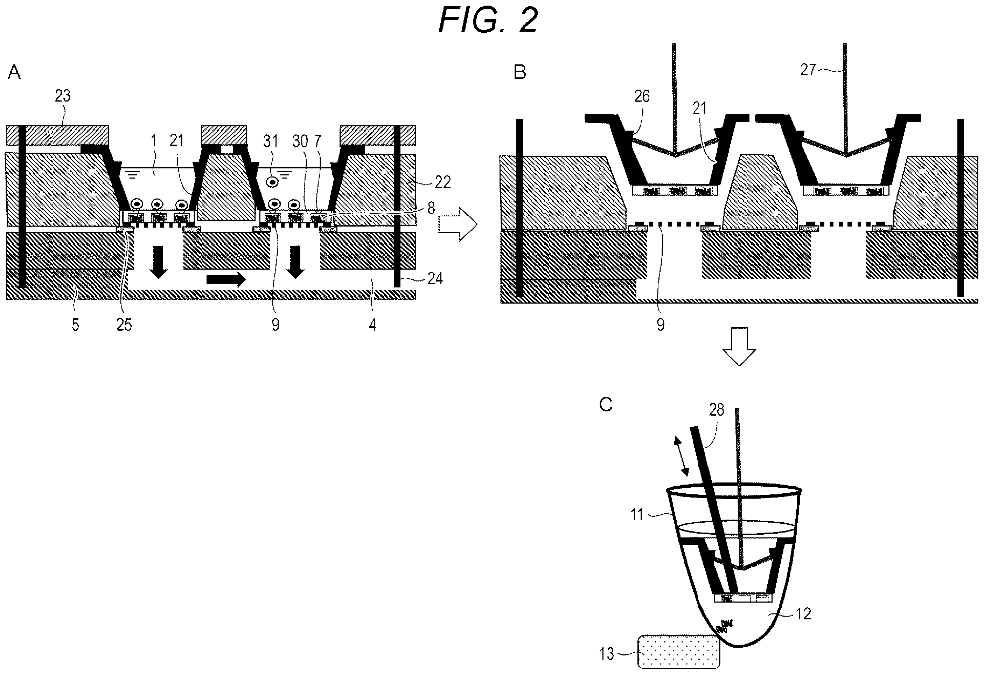

FIG. 2 illustrates a specific structural example of a container for single cell analysis according to the present invention and how to use the container. The lower plate 5 on which the aspiration flow path 4 may be arranged is the same as the conventional device (FIG. 1). The difference is that the bottom surface of the container 21 for storing the cell suspension 1 may be fixed so as to be coincide with the single cell analysis chip 30. A membrane 9 for holding beads may be in close contact with the lower side of the single cell analysis chip. A sealing rubber that prevents air leakage when a negative pressure is applied using an aspiration pump may be inserted between the sealing rubber 25 and the lower plate. Further, to prevent air leakage, the container 21 must be arranged to match an opening of the lower plate, and pressure must be applied toward the lower plate. This is achieved by an upper plate 23, an intermediate plate 22, and a positioning pin 24. Positioning pins may be passed through holes formed in 23 and 22, thereby enabling in-plane positioning with respect to the lower plate 5. In addition, screws may be fastened onto screw pits formed in 5 with respect to 23, and thus the upper plate may be brought into close contact with the lower plate, and as a result air leakage does not occur.

As a method of using the single cell analysis device, a pump may be connected to the flow path 4, and cells 31 in the cell suspension 1 may be aspirated and trapped by the cell trapping portion 7 and isolated, which is the same as the conventional method (FIG. 1). Further, the procedures of the reverse transcription reaction, subsequent cooling, and removal of the reverse transcriptase solution may be the same as the conventional method.

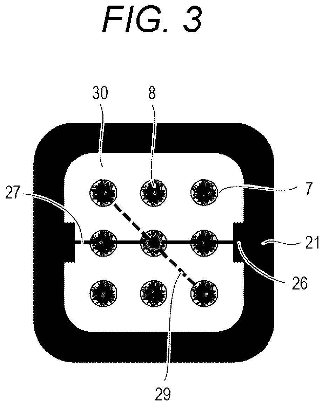

Thereafter, the screws fixing the upper plate 23 may be loosened, and the upper plate 23 may be removed. Then, a container lifting rod 27 may be hooked on rod fixing projections 26 and the container 21 may be lifted. FIG. 3 illustrates a structural view from above when a container for single cell analysis 21 is lifted from the single cell analysis device. A bottom of the container 21 may be the single cell analysis chip 30. The rod 27 may be inserted into the interior of the container at an angle 29 (state indicated by dotted line in FIG. 3). The rod 27 may be rotated to a position 27 and lifted, and thus the container 21 can be hooked and fixed onto the rod fixing projections 26.

The container 21 lifted by the rod 27 may be immersed in the bead suspension and collection buffer 12 in the tube 11. In addition, vibration may be applied to the single cell analysis chip 30 by a vibration bar 28. The container may be pressed against the inner wall of the tube to achieve firm fixation, and thus the chip 30 may be firmly fixed, and the vibration of the vibration bar can be efficiently applied to the chip 30.

As a result, it is possible to diffuse the beads of the nucleic acid trapping portion into the solution in a short time and collect the beads using the magnet 13.

In another aspect, the present invention relates to a single cell analysis method using a single cell analysis device, in the single cell analysis device to which the container for single cell analysis according to the present invention is fixed, the method including the steps of:
introducing a solution containing cells into the container;
trapping the cells in the cell trapping portion;
reacting nucleic acid derived from the trapped cells in the microreaction chamber;
removing the container from the single cell analysis device after the reaction; and
collecting the solid phase from the removed container.

In one embodiment, the step of collecting the solid phase may include immersing the container for single cell analysis in another container containing a solution and vibrating the container for single cell analysis. For example, when the solid phase is a magnetic bead, the single cell analysis method according to the present invention may include collecting the solid phase (magnetic bead) in the other container by a magnet, with the vibration or after the vibration.

**In** another aspect, the present invention relates to an automated single cell analysis device including:
a single cell analysis device configured to fix the container for single cell analysis according to the present invention;
a mechanism that is configured to remove the container from the single cell analysis device and move the container to another container;
a mechanism that is configured to vibrate the container in the other container; and
a control device,
in which the control device is configured to remove the container from the single cell analysis device after a reaction in the single cell analysis device, and to control a movement of the container to the other container and a vibration of the container in the other container.

**In** one embodiment, the control device may be configured to control a fixture fixing the container for single cell analysis to the single cell analysis device to be removed. **In** one embodiment, the control device may be configured to control the container for single cell analysis to be pressed against the single cell analysis device to prevent air leakage when aspirating a reaction solution from the container for single cell analysis.

The container for single cell analysis according to the present invention and the single cell analysis method using the same may enable quick and easy collection of a solid phase on which nucleic acid (mRNA) derived from cells is immobilized after a reaction in the single cell analysis device, and may particularly be useful in the automated device as described above. Further, it is possible to prevent contamination of substances other than the desired solid phase and perform more accurate single cell analysis.

### Examples

Hereinafter, the present invention will be specifically described with reference to Examples, but these Examples are merely provided for the purpose of describing the present invention, and do not limit or restrict the scope of the invention disclosed in this application.

### [Example 1]

In this Example, the structure of the single cell analysis device including the container for single cell analysis having the single cell analysis chip as the bottom surface as well as the single cell analysis method using the same will be described.

(1) With regard to structure of single cell analysis device including container for single cell analysis having single cell analysis chip as bottom surface

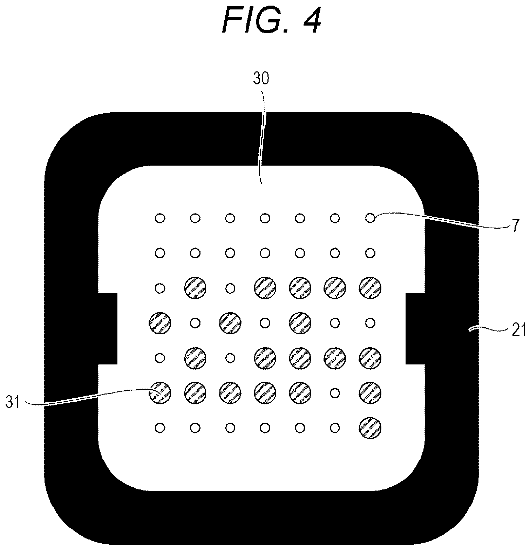

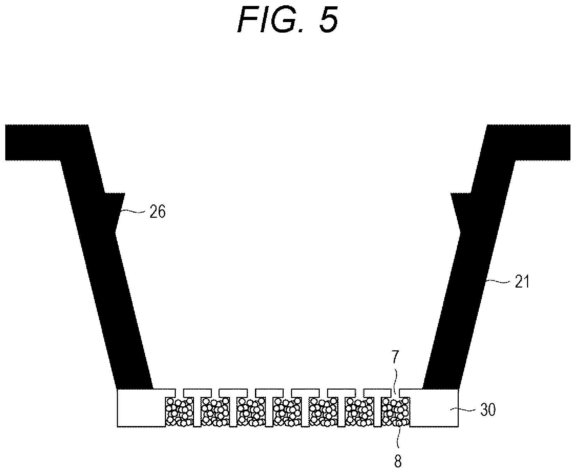

FIG. 4 illustrates the container for single cell analysis 21 in the case of this example, as viewed from above. Here, the cell suspension is contained in the container, and a negative pressure is applied from the back surface, and thus the solution is aspirated from the cell trapping portion 7, and the cells 31 are trapped and isolated. FIG. 5 is a cross-sectional view of the container for single cell analysis 21 in a case where no cell suspension is contained. The nucleic acid trapping portion 8 filled with beads is arranged immediately below the cell trapping portion 7, and a flow path penetrating the single cell analysis chip 30 is formed.

Here, the thickness of the single cell analysis chip was about 100 µm, and the diameter of the cell trapping portion was about 2 to 5 µm. Further, the size of the nucleic acid trapping portion was a cylindrical shape having a diameter of 75 µm and a depth of 70 µm. This region is filled with magnetic beads at a filling rate of 50% to 95%. In the filling method, a poly T sequence-containing RT probe (CCATCTCATCCCTGCGTGTCTCCGACTCAGTCGCGTACNNNNNNNTTTTTTTT TTTTTTTTTTVN: SEQ ID NO: 1) for trapping mRNA is immobilized on beads (1 µm in diameter), and a cell identification tag (as an example of about 100 known sequences, TCGCGTAC) and a molecular identification tag sequence (NNNNNNNN, N = A, G, C, or T) are also inserted into this RT probe as described in WO 2016/125251 A. The treatment method from dispensing of cells to the reverse transcription reaction is shown below.

In addition to PDMS, a resin material used for the enzyme reaction in the biotechnology, such as polycarbonate, polypropylene, polyethylene, polypropylene, or cycloolefin can be used as the material of the chip.

The size of the chip was 3.4 mm square, and a single cell analysis well was attached to the chip using an adhesive. The size of the well was 4.5×4.5 mm square at the largest upper portion and 3.5 mm in height. The material is acryl, but a different material such as polycarbonate, polypropylene, or cycloolefin may be used.

In addition, a 0.8 µm-pore track etch membrane for flowing a solution only in a direction perpendicular to the membrane and having a porous diameter smaller than the diameter of the beads was used as the porous membrane for holding the beads. This track etch membrane is in close contact with the PDMS chip, thereby preventing the beads from leaking.

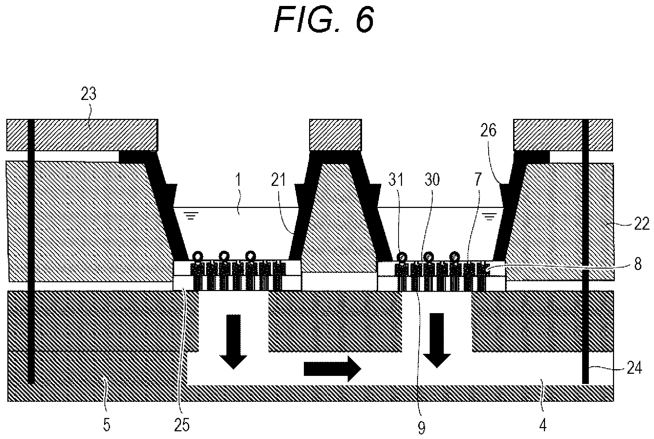

FIG. 6 illustrates a cross-sectional view of the single cell analysis device in this example. The names and functions of the portions are similar to those in FIG. 2. Although acryl is used as the material of the device, a different resin material may be used similarly to the case of the container for single cell analysis.

Further, in this example, 2 chips are connected to the 2 aspiration flow paths 4. In the device, 2 aspiration flow paths (one of the flow paths is arranged on the back side in FIG. 6) are provided and 4 chips are mounted on one device. However, the number of chips to be mounted can be increased by increasing the area of the device. In a case where one device is designed to treat one kind of specimen and the number of single cells to be analyzed is about 470, 12 chips may be mounted on the device. In a case where 940 single cells are required, 24 chips may be mounted (it is possible to analyze cells of about 80% of the cell trapping portion). Further, in this example, 7×7 cell trapping portions and nucleic acid trapping portions corresponding thereto were provided in one chip. Setting the number to 10×10, 20×20, 30×30, and 100×100 makes it possible to treat about 80, 320, 720, and 8000 cells on one chip, respectively. Preparing a device with 12 chips mounted thereon makes it possible to perform single cell analysis on 960, 3840, 8640, and 96000 cells on one device.

### (2) Trapping of cells and reverse transcription reaction from cells using single cell analysis device

In the state of FIG. 6 in which the container for single cell analysis 21 is fixed to the single cell analysis chip (VFACs) 30, the cell suspension 1 is dispensed into the container, and the aspiration pump is connected to the aspiration flow path 4. The aspiration pump is switched on, and the pump aspiration (90 kPa) is performed from the lower direction of the VFAC, whereby the individual cells 31 are trapped by the 3 µm cell trapping portion 7 on the VFACs. Subsequently, 8 µL of cell wash buffer (100 mM Tris (pH 8.0), 500 mM NaCl, 5 mM DTT, 0.4 U/µL RNase OUT, 0.1% Tween 20) is added, and pump aspiration (90 kPa) is performed for re-trapping a small number of cells remaining on the surface of the VFACs or the like. 1 µL of cell lysis buffer (100 mM Tris (pH 8.0), 500 mM NaCl, 10 mM EDTA, 1% SDS, 5 mM DTT, 1.33 U/µL RNase OUT) is added to lyse the cells, and the eluted mRNA is trapped by the bead-immobilized RT probe. After the reaction, the reagents are removed by performing pump aspiration (90 kPa). After addition of 8 µL of lysis wash buffer (100 mM Tris (pH 8.0), 500 mM NaCl, 5 mM DTT, 0.4 U/µL RNase OUT, 1% Tween 20), pump aspiration (90 kPa) is performed to remove the residual reagents in the cell lysis buffer that can cause inhibition of enzyme reaction. This operation is repeated twice. It has been confirmed that no loss of cell-derived mRNA (about 10⁶ molecules/cell) occurs even when a series of pump aspiration operations is performed at a strength of 90 kPa in this step. Both the cell lysis buffer and the lysis wash buffer contain NaCl at a high concentration of 500 mM, and a sufficient amount (1.5×10¹⁰ molecules) of the RT probe is immobilized on the magnetic beads. Thus, a trace amount of intracellular mRNA is efficiently trapped. To the upper surface of each of the VFACs, 4.5 µL of reverse transcription reaction reagent (1×FS Buffer (Takara Bio Inc.), 2 mM DTT, 2 mM dNTPs, 3.2 U/µL RNase inhibitor (Takara Bio Inc.), 10 Unit/µL SmartScribe RT (Takara Bio Inc.), 0.2% Tween20) is added. The opening on the upper surface of the single cell analysis device is sealed with an optical adhesive film (manufactured by Thermo Fisher Scientific, Inc.), and incubated in a thermostat bath heated to 42°C in advance for 60 minutes. After 5 minutes of incubation at room temperature, the seal of the device is peeled off and the reverse transcription reaction reagent is aspirated off with the pump.

A 100 µL tube is put into a 0.5 mL tube previously charged with a suspension buffer (50 mM NaCl, 50 mM Tris (pH 8.0)).

Thereafter, the screws fixing the upper plate are loosened, and the upper plate 23 is removed. Then, a container lifting rod 27 is hooked on rod fixing projections 26 and the container 21 is lifted.

The container 21 lifted by the rod 27 is immersed in the tube containing the bead suspension and collection buffer 12. In addition, vibration is applied to the single cell analysis chip 30 by a vibration bar 28. As the vibration bar, a carbonate-based bar (rod) having a diameter of about 1 mm was fixed to a commercially available pen-type vibrator and used. The container 21 was vibrated while the rod was pressed against the chip portion on the lower side of the inner wall of the tube. As a result, it was possible to suspend the beads in the solution in about 2 to 3 seconds per chip. A neodymium magnet was brought close to the bottom of the tube, and the beads were collected. Thereafter, the container 21 is removed, and the beads are washed twice with 50 µL of wash buffer (0.1% Tween 20, 10 mM Tris, pH 8.0). Then, the bead suspension is transferred to a 0.2 mL PCR tube, and the beads are suspended in 1 µL of the same suspension.

### (3) Preparation of sample for analysis with next generation sequencer (NGS) based on PCR amplification

### (3-1) 1st multiplex PCR amplification

The RT probe on the magnetic beads is degraded with Exonuclease I, and then the sample prepared in (2) is subjected to 1st multiplex PCR amplification. Here, a Forward primer set mixed with 44 kinds of oligos (SEQ ID NOs: 9 to 52) described in Table 1 below and one kind of Reverse primer (PA primer, CCATCTCATCCCTGCGTGTCT: SEQ ID NO: 2) were used.

**[Table 1]**

| No. | Primer name | Sequence (5' to 3') | SEQ ID NO: | Base length (nt) |
|---|---|---|---|---|
| 1 | Acaca | TCTCTGGCCTCCACTTTTGCT | 9 | 21 |
| 2 | Batf | GAGGTGGTATACAGTGCCCAT | 10 | 21 |
| 3 | Bc12 | AGGATTGATGGCAGATTCAGT | 11 | 21 |
| 4 | Bc1-6 | GACTATTTTAAGTATTGCGTCTGT | 12 | 24 |
| 5 | Bt1a | CCCTTTTCTATTGCACTCCAC | 13 | 21 |
| 6 | Cd160 | ATGAACAGAGAAGTCATTTGCC | 14 | 22 |
| 7 | Cd244 | GCCAGATGTCAGTCTTGTTCAC | 15 | 22 |
| 8 | Cd3e | GCTAAGCCCTTTCCTACAGC | 16 | 20 |
| 9 | Cd4 | TTCTCTTAGAGTGAGGCTGGG | 17 | 21 |
| 10 | Cd8a | TGGAGAACATTCCTTAGCACCC | 18 | 22 |
| 11 | Cd8b1 | CACAGAGTGGCTGAAGAACCA | 19 | 21 |
| 12 | Clla4 | GGCTCAGTTGCATAAACCGAT | 20 | 21 |
| 13 | Cxcr3 | CGCAGCCCAAGTCCTAACACA | 21 | 21 |
| 14 | Eomes | TTGTTGTAGGGCTGGCTCTGT | 22 | 21 |
| 15 | Fas | GAGAGAGCCTGCCACCCAT | 23 | 19 |
| 16 | Fasl | TACAAGGGTGAGAAAGGAGGC | 24 | 21 |
| 17 | Foxp3 | CTATGCCCCTATAAGACCACCCT | 25 | 23 |
| 18 | Gzmb | GCAGGCCAATGGAACACCTC | 26 | 20 |
| 19 | Havcr2 | AATATTCTTTGGGAGGACAGTCG | 27 | 23 |
| 20 | Hif-1a | TGCAGTATGAATGGAGTAAGTGA | 28 | 23 |
| 21 | lcos | CCTCTAGTCTTTGGTCTGCAT | 29 | 21 |
| 22 | lfng | CTGACTAATTAGCCAAGACTGTG | 30 | 23 |
| 23 | ll10 | TTTAAGCTGTTTCCATTGGGG | 31 | 21 |
| 24 | ll17a | AGAATTGTCTGCCCTCCACA | 32 | 20 |
| 25 | ll2rb | ACGAAGAGGTCCTGATAAGACTG | 33 | 23 |
| 26 | ll2rg | TCCTAAGTGACGCTAACCTCC | 34 | 21 |
| 27 | ll7r | GTCACAATTTTGAATCCCTGCTG | 35 | 23 |
| 28 | Klrg1 | TCATGTGCTCACTCCTGCTT | 36 | 20 |
| 29 | Lag3 | GTACGCCGCAGAGTCTAGCTCA | 37 | 22 |
| 30 | Mki67 | AAACTAGAGTCGCTTTGGACAG | 38 | 22 |
| 31 | Pdcd1 | TGATGAAGACTTGAAAAGCTCCT | 39 | 23 |
| 32 | Prdm1 | GCAGCCCCAACAAGACTGACA | 40 | 21 |
| 33 | Prf1 | CCTACCATGCCAAGTGTCTGC | 41 | 21 |
| 34 | Rorc | TCCTGAGACTTTTCTGCCTTTC | 42 | 22 |
| 35 | Runx3 | CAGTGAGCACAGGCAGCTGGG | 43 | 21 |
| 36 | Slc2a1 | CCTCGGCACCATAGGGGTC | 44 | 19 |
| 37 | Tbp | GCAGCACTACTGTGAGTTGCTT | 45 | 22 |
| 38 | Tbx21 | GGTCGGGGTGGGGAGTCCAG | 46 | 20 |
| 39 | Tgfb | TGCCAACTTCTGTCTGGGACCCT | 47 | 23 |
| 40 | Tigit | CTGTGCTGGGACTCATTTGCT | 48 | 21 |
| 41 | Tnf | GAGGCTGGATAAGATCTCAGG | 49 | 21 |
| 42 | Tnfrsf9 | TTTGAGGGGAATCTTCAGAGC | 50 | 21 |
| 43 | Vsir | TGCCTTGGACTCAGGAAGGGAA | 51 | 22 |
| 44 | Zeb2 | GAGACATTTGTGATGTTGGCTGT | 52 | 23 |

Details of the 1st multiplex PCR amplification are as follows. An Exonuclease I reaction solution (1 x Exonuclease I buffer, 0.067 unit/µL) is prepared on ice. 14 µL of the reaction solution is mixed with the sample prepared in (2) and the mixture is incubated at 37°C for 15 minutes. The beads are washed 3 times with 50 µL of wash buffer (0.1% Tween 20, 50 mM Tris, pH 8.0), and then the beads are suspended in 1 µL of 10 mM Tris (pH 8.0). 9 µL of 1st multiplex PCR reagent (1 x Gflex PCR buffer, 0.2 µM 44 plex primer mix, 2 µM PA primer, 0.075 unit/µL Tks Gflex DNA polymerase) is prepared on ice. 9 µL of the prepared 1st multiplex PCR reagent is mixed with 1 µL of the bead sample, and the resultant mixture is subjected to PCR amplification under appropriate temperature conditions (after heat deactivation at 94°C for 1 minute, 14 cycles at 98°C for 10 seconds, at 58°C for 3 minutes, and at 68°C for 25 seconds, and at 68°C for 2 minutes, and then at a constant temperature of 4°C). After the beads are trapped by a neodymium magnet, 10 µL of the supernatant containing the amplification product is collected in a tube. The beads are washed with 40 µL of wash buffer (0.1% Tween 20, 10 mM Tris, pH 8.0), and the supernatant is mixed with the amplification product to make a total of 50 µL. 35 µL of Ampure XP (volume of sample x 0.7) is added to and mixed with the mixture. The resultant mixture is purified in accordance with the manufacturer's recommended protocol, and finally eluted with 35 µL of wash buffer (10 mM Tris (pH 8.0), 0.1% Tween20), and the supernatant is collected in another tube.

### (3-2) 2nd multiplex PCR amplification

The 1st multiplex PCR product (gene analysis product) obtained in (3-1) is subjected to 2nd multiplex PCR amplification using a set of 44 kinds of Forward primers (Table 2, SEQ ID NOs: 53 to 96) to which R2SP (common sequence for NGS, manufactured by Illumina, Inc.) is added and one kind of Reverse primer (PAprimer, SEQ ID NO: 2). As a result, DNA for gene expression analysis is amplified.

**[Table 2]**

| No. | Primer name | Sequence (5' to 3') | SEQ ID NO: | Base length (nt) |
|---|---|---|---|---|
| 1 | Acaca | GTGACTGGAGTTCAGACGTGTGCTCTTCCGA TCTCCCCGGTG GTATGAAAGCTG | 53 | 54 |
| 2 | Batf | GTGACTGGAGTTCAGACGTGTGCTCTTCCGA TCTTGCCTTCCA CCAGCCTCACATCA | 54 | 57 |
| 3 | Bcl2 | GTGACTGGAGTTCAGACGTGTGCTCTTCCGA TCTTTTCCTTTG GAGACAGGGGTTCTCTA | 55 | 60 |
| 4 | Bcl-6 | GTGACTGGAGTTCAGACGTGTGCTCTTCCGA TCTTTTGTCTAA AATGCCTCCGTGT | 56 | 56 |
| 5 | Btla | GTGACTGGAGTTCAGACGTGTGCTCTTCCGA TCTTCTCTCATT GAACCTGGAGCTCTG | 57 | 58 |
| 6 | Cd160 | GTGACTGGAGTTCAGACGTGTGCTCTTCCGA TCTCAACACCAC ATCCAAATACAGGG | 58 | 57 |
| 7 | Cd244 | GTGACTGGAGTTCAGACGTGTGCTCTTCCGA TCTTCCTTCATC CCTGTACATGCCTAG | 59 | 58 |
| 8 | Cd3e | GTGACTGGAGTTCAGACGTGTGCTCTTCCGA TCTTTTTATTCC CTTCCCGGCCCAT | 60 | 56 |
| 9 | Cd4 | GTGACTGGAGTTCAGACGTGTGCTCTTCCGA TCTGTGCAGAG CCTGGGAAGTGGA | 61 | 55 |
| 10 | Cd8a | GTGACTGGAGTTCAGACGTGTGCTCTTCCGA TCTTGTAAAGAG GCAGACTAGACGTGG | 62 | 58 |
| 11 | Cd8b1 | GTGACTGGAGTTCAGACGTGTGCTCTTCCGA TCTGACTGGGC CTTGGACAGTGG | 63 | 54 |
| 12 | Clla4 | GTGACTGGAGTTCAGACGTGTGCTCTTCCGA TCTATGGTGAT GGAGTGTAAACTGGG | 64 | 57 |
| 13 | Cxcr3 | GTGACTGGAGTTCAGACGTGTGCTCTTCCGA TCTAGTGCTTGT CCTCCTTGTAGTTGGG | 65 | 59 |
| 14 | Eomes | GTGACTGGAGTTCAGACGTGTGCTCTTCCGA TCTAAATTTGGT GCTTTGGCGTTGTAA | 66 | 58 |
| 15 | Fas | GTGACTGGAGTTCAGACGTGTGCTCTTCCGA TCTATTGACATT GGCAACTCCTGGTG | 67 | 57 |
| 16 | Fas1 | GTGACTGGAGTTCAGACGTGTGCTCTTCCGA TCTCAGTGTCTC ATTGGCACCATC | 68 | 55 |
| 17 | Foxp3 | GTGACTGGAGTTCAGACGTGTGCTCTTCCGA TCTCAACAGTG CACCTAAAGGACTCA | 69 | 57 |
| 18 | Gzmb | GTGACTGGAGTTCAGACGTGTGCTCTTCCGA TCTACCATGCTG TGACAACCCAACTGA | 70 | 58 |
| 19 | Havcr2 | GTGACTGGAGTTCAGACGTGTGCTCTTCCGA TCTGACCTCAGT TTCATCTGGCTT | 71 | 55 |
| 20 | Hif-1a | GTGACTGGAGTTCAGACGTGTGCTCTTCCGA TCTATCTGTTCC CATTAGCAGGTGA | 72 | 56 |
| 21 | lcos | GTGACTGGAGTTCAGACGTGTGCTCTTCCGA TCTATCTTCCCC TGGCTTCTAGGATGTATG | 73 | 61 |
| 22 | lfng | GTGACTGGAGTTCAGACGTGTGCTCTTCCGA TCTGATTGCGG GGTTGTATCTGGG | 74 | 55 |
| 23 | ll10 | GTGACTGGAGTTCAGACGTGTGCTCTTCCGA TCTTTATATGAT GGGAGGGGTTCTTC | 75 | 57 |
| 24 | ll17a | GTGACTGGAGTTCAGACGTGTGCTCTTCCGA [?] | 76 | 55 |
| | | TCTGAAGGTGT AAAGGGGTCCCAA | | |
| 25 | ll2rb | GTGACTGGAGTTCAGACGTGTGCTCTTCCGA TCTCTCGTAGAA GGAGGGGTTGGG | 77 | 55 |
| 26 | ll2rg | GTGACTGGAGTTCAGACGTGTGCTCTTCCGA TCTCCAATGCTC ACTGCCTTCCCT | 78 | 55 |
| 27 | ll7r | GTGACTGGAGTTCAGACGTGTGCTCTTCCGA TCTGATACTGTG GTTGGGTGCCTT | 79 | 55 |
| 28 | Klrg1 | GTGACTGGAGTTCAGACGTGTGCTCTTCCGA TCTCTTGTGCCT GAGCTGTAGGGA | 80 | 55 |
| 29 | Lag3 | GTGACTGGAGTTCAGACGTGTGCTCTTCCGA TCTACCTTAAAG GAGGCCATCTCGT | 81 | 56 |
| 30 | Mki67 | GTGACTGGAGTTCAGACGTGTGCTCTTCCGA TCTACCTAAGCC AAGGGTAACTCG | 82 | 55 |
| 31 | Pdcd1 | GTGACTGGAGTTCAGACGTGTGCTCTTCCGA TCTCCGTGAGC GTATCTGCTGTCC | 83 | 55 |
| 32 | Prdm1 | GTGACTGGAGTTCAGACGTGTGCTCTTCCGA TCTCCAAAGCAT GACCTGACATTGACACC | 84 | 60 |
| 33 | Prf1 | GTGACTGGAGTTCAGACGTGTGCTCTTCCGA TCTCGCAGTGG GGCTGTGTGGTAA | 85 | 55 |
| 34 | Rorc | GTGACTGGAGTTCAGACGTGTGCTCTTCCGA TCTCCTCTGCCT GTTTTCTGGGAC | 86 | 55 |
| 35 | Runx3 | GTGACTGGAGTTCAGACGTGTGCTCTTCCGA TCTAAAGGGTC CTTATTTGCAAGGCTAC | 87 | 59 |
| 36 | Slc2a1 | GTGACTGGAGTTCAGACGTGTGCTCTTCCGA TCTTTAGGATTC GCCCATTCCTGTC | 88 | 56 |
| 37 | Tbp | GTGACTGGAGTTCAGACGTGTGCTCTTCCGA TCTCTTGCTCTG TGCTGCTACTTGGG | 89 | 57 |
| 38 | Tbx21 | GTGACTGGAGTTCAGACGTGTGCTCTTCCGA TCTAGCCAAACA GAGAAGACTCAGGTGAC | 90 | 60 |
| 39 | Tgfb | GTGACTGGAGTTCAGACGTGTGCTCTTCCGA TCTGGACCCTG CCCCTATATTTGGAG | 91 | 57 |
| 40 | Tigit | GTGACTGGAGTTCAGACGTGTGCTCTTCCGA TCTCTATAGAAA GTGGCCTTGGGAG | 92 | 56 |
| 41 | Tnf | GTGACTGGAGTTCAGACGTGTGCTCTTCCGA TCTGCAATGCA CAGCCTTCCTCACA | 93 | 56 |
| 42 | Tnfrsf9 | GTGACTGGAGTTCAGACGTGTGCTCTTCCGA TCTGCCTGCTTC TCCTGTTTCCTC | 94 | 55 |
| 43 | Vsir | GTGACTGGAGTTCAGACGTGTGCTCTTCCGA TCTCTTCCCAAA AGATTTCCACGTGTGT | 95 | 59 |
| 44 | Zeb2 | GTGACTGGAGTTCAGACGTGTGCTCTTCCGA TCTAATCTTTGT TACCTTCGCTGTGA | 96 | 57 |

Details of the 2nd multiplex PCR amplification are as follows. 13 µL of 2nd multiplex PCR reagent (1 x Multiplex PCR Plus, 0.3 µM 2nd R2SP-added 44plex primer mix, 3 µM PA primer) is mixed with 7 µL of the 1st multiplex PCR product, and then the mixture is amplified under appropriate temperature conditions (after heat deactivation at 95°C for 5 minutes, 3 cycles at 95°C for 30 seconds, at 61°C for 5 minutes, and at 72°C for 30 seconds, then 3 cycles at 95°C for 30 seconds, at 59°C for 5 minutes, and at 72°C for 30 seconds, then 3 cycles at 95°C for 30 seconds, at 57°C for 5 minutes, and 72°C for 30 seconds, 3 cycles at 95°C for 30 seconds, at 55°C for 5 minutes, and at 72°C for 30 seconds are performed, at 72°C for 2 minutes, and then at a constant temperature of 4°C). To 20 µL of the 2nd multiplex PCR product, 30 µL of 0.1% Tween 20 (10 mM Tris, pH 8.0) is added (50 µL in total). 35 µL of Ampure XP (volume of sample x 0.7) is added to and mixed with the mixture. The resultant mixture is purified in accordance with the manufacturer's recommended protocol, and finally eluted with 40 µL of wash buffer (10 mM Tris (pH 8.0), 0.1% Tween20), and the supernatant is collected in another tube.

### (3-3) 3rd PCR for NGS library preparation

3rd PCR amplification is performed using the 2nd multiplex PCR product obtained in (3-2) as a template, thereby introducing a common sequence (P5-R1SP (AATGATACGGCGACCACCGAGATCTACACTCTTTCCCTACACGACGCTCTTC CGATCT: SEQ ID NO: 3), P7-R2SP (CAAGCAGAAGACGGCATACGAGATGTGACTGGAGTTCAGACGTGTGCTCTT CCGATCT: SEQ ID NO: 4)) necessary for next-generation sequencing (NGS) analysis, manufactured by Illumina, Inc., and a Chip Tag (CT) sequence (as an example of 64 known sequences, TGACATA) for identifying VFAC. Detailed conditions are shown below.

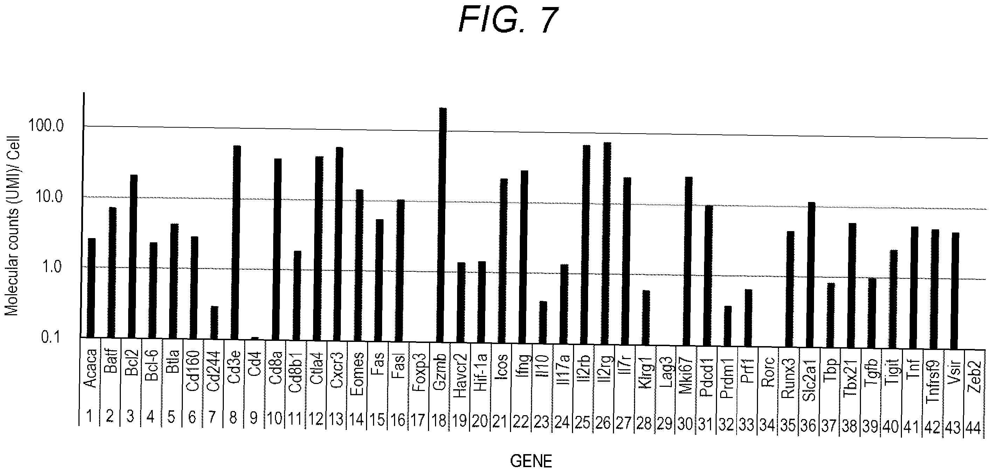

22 µL of 3rd PCR reagent (1 x Gflex PCR buffer, 0.23 µM P5_R1SP_CT_PA primer (as an example of 64 kinds of CT, AATGATACGGCGACCACCGAGATCTACACTCTTTCCCTACACGACGCTCTTCC GATCTTGACATACCATCTCATCCCTGCGTGTCTC: SEQ ID NO: 5), 0.23 µM P7_R2SP primer (CAAGCAGAAGACGGCATACGAGATGTGACTGGAGTTCAGACGTGT: SEQ ID NO: 6), 0.01 µM P5 primer (AATGATACGGCGACCACCGAGATCTACAC: SEQ ID NO: 7), 0.01 µM P7 primer (CAAGCAGAAGACGGCATACGAGAT: SEQ ID NO: 8), 0.045 Unit/µLTks Gflex DNA polymerase) is prepared on ice, and mixed with 8 µL of the 2nd multiplex PCR product obtained in (3-2). Amplification is performed under appropriate temperature conditions (after heat deactivation at 94°C for 1 minute, 1 cycle at 98°C for 10 seconds, at 61°C for 1 minute, and at 68°C for 25 seconds, 2 cycles at 98°C for 10 seconds, at 59°C for 1 minute, and at 68°C for 25 seconds, 2 cycles at 98°C for 10 seconds, at 57°C for 1 minute, and at 68°C for 25 seconds, 2 cycle at 98°C for 10 seconds, at 55°C for 1 minute, and at 68°C for 25 seconds are performed, at 68°C for 2 minutes, and then at a constant temperature of 4°C). 20 µL of 0.1% Tween 20 (10 mM Tris, pH 8.0) is added to 30 µL of the amplification product for diluting to 50 µL in a measuring flask. Thereafter, 35 µL of Ampure XP (volume of sample x 0.7) is added to and mixed with the mixture. The resultant mixture is purified in accordance with the manufacturer's recommended protocol, and finally eluted with 40 µL of wash buffer (10 mM Tris (pH 8.0), 0.1% Tween20), and the supernatant is collected in another tube. 1 µL of the 3rd PCR amplification product is analyzed by chip electrophoresis, the size (bp) and concentration (pmol/L) of the amplification product are determined. Then, NGS analysis (Miseq, manufactured by Illumina, Inc.) is performed. The data after the NGS analysis is separated for each tag sequence (chip tag, cell identification tag, molecular identification tag) and mapped on the sequences published on a public database of 44 genes. Thus, the number of molecules of mRNA can be counted and gene sequence data for each cell can be acquired. The results are shown in FIG. 7. As shown in the results, it is shown that gene expression analysis derived from single cells can be performed by using the container for single cell analysis of this example.

### [Example 2]

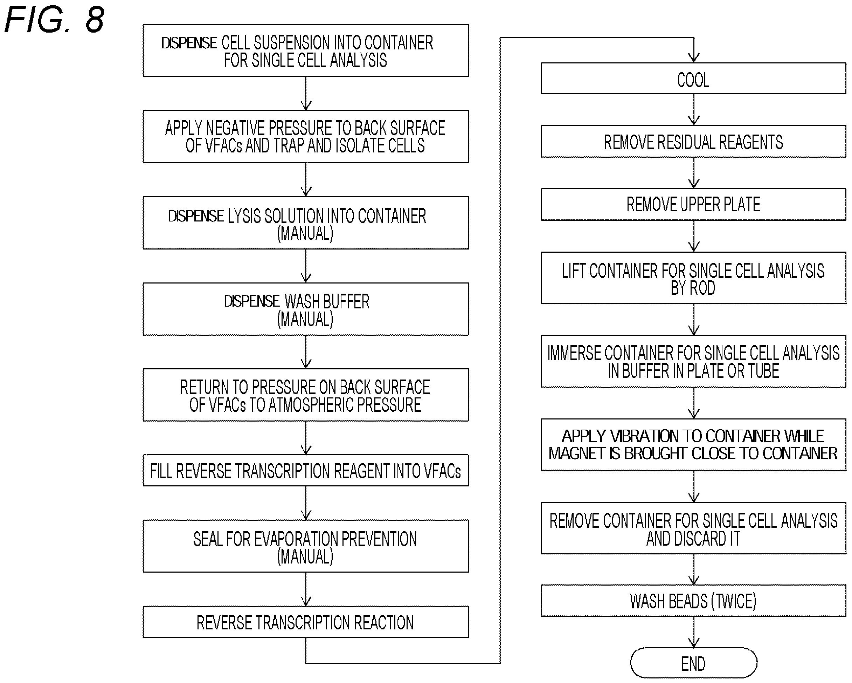

This example describes a device in which steps up to collection of beads in the single cell analysis using the container for single cell analysis are automated. FIG. 8 shows a flowchart of an automated process.

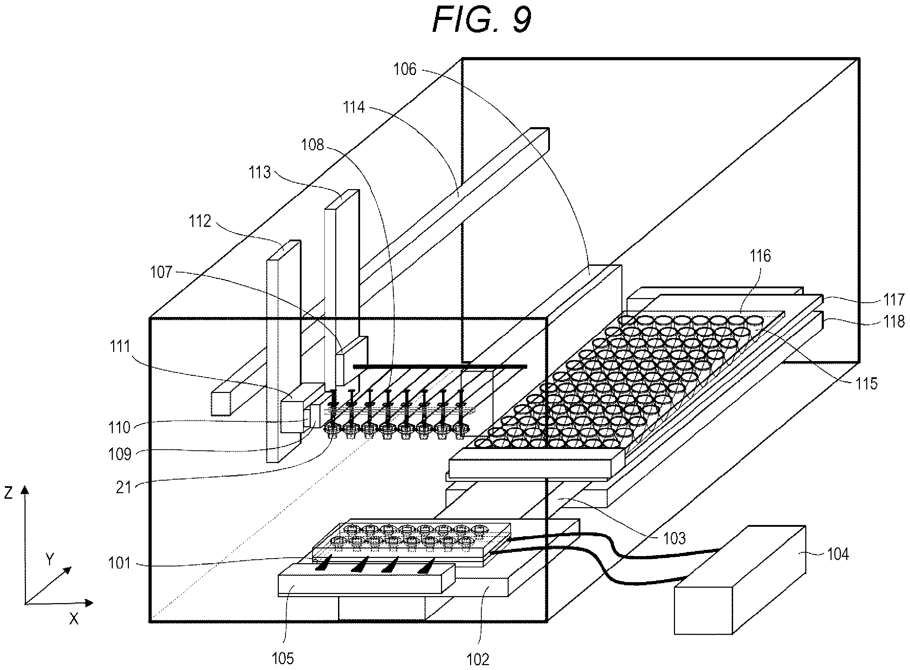

Subsequently, a function of a system that realizes automation of the steps will be described with reference to the view of the configuration of the device illustrated in FIG. 9. First, a device 101 similar to the single cell analysis device used in Example 1 is fixed on an XY-axes movable stage 102. However, in the single cell analysis device 101 fixed here, the lower plate is fixed to the stage in a state where the upper plate is not fixed with screws (a state where the no screw is attached) for automation. At this time, a cell suspension containing cells to be analyzed is dispensed in advance into the container for single cell analysis 21. When the program is started to start the step, the single cell analysis chip is pressed against the lower plate. An XZ-axes movable stage 102 and a Y-axis movable stage 103 are moved such that the upper plate is pressed against a blade 105 (black triangle parts of the blade 105) for removing the upper plate from below to prevent air leakage during aspiration. At this time, the position of the single cell analysis device is adjusted using the XZ-axes movable stage such that the opening of the single cell analysis well is not hidden by the blade. The blade has a triangular plate-like shape. The blade was formed using a 3-mm-thick member of an SUS material such that the blade would not bend even when the required pressing pressure of the single cell analysis device was applied. The intensity of the pressure was monitored by arranging a pressure sensor on the lower side of the single cell analysis device. In this state, a desired pressure is maintained, aspiration by an aspiration pump 104 is started, and cells are isolated. It is visually confirmed that the cell suspension has been removed from the container, and a lysis buffer and a lysis wash solution are introduced in a similar manner to Example 1. After it is confirmed that the solution has been removed, the pump is turned off. In this example, the solution is dispensed by manual operation, but a known robotic dispensing mechanism can also be used.

After it is confirmed that the pressure has returned to the atmospheric pressure, the reverse transcriptase is manually dispensed into the container in a similar manner to Example 1. The pump is turned on for a short time of about 2 seconds, whereby the nucleic acid trapping portion (microreaction chamber) of the single cell analysis chip can be filled with the solution (reverse transcription reagent). It is confirmed that the enzyme solution remains in each container, and the opening is sealed manually. Thereafter, a plate-like heater 102 arranged under the single cell analysis device on the XZ-axes movable stage is turned on to raise the temperature of the device to 42°C in a similar manner to Example 1, thereby causing a reverse transcription reaction. After completion of the reaction time, the device is cooled to room temperature, then the seal is removed, the pump is switched on, and the reagent (enzyme solution) remaining in the container is aspirated.

Next, to remove the upper plate, the XZ-axes movable stage 102 is once lowered to set the pressure to 0. Then, the XZ-axes movable stage 102 and the Y-axis movable stage 103 are controlled such that the blade is inserted between the upper plate and the intermediate plate. After insertion, the Z-axis movable stage is further lowered, and the container for single cell analysis and the intermediate plate are separated from the upper plate.

Next, the stages 102 and 103 are used to move the single cell analysis device to a region accessible by the container lifting rod. The container lifting rod connected to stages 113 and 114 and connected to a pickup arm 107 for placing the container lifting rod in an appropriate position is lowered, and hooked on the projections of the container for single cell analysis. Here, a screw structure is formed in the projections 26 such that the rod is rotated only by pushing the rod for hooking the rod. Then, the rod is lifted using the stages 113 and 114, moved to just above a 96-well plate 115, and the container is immersed in the previously dispensed buffer. After that, while the immersed state is maintained, a vibration bar 108 is brought into contact with the single cell analysis chip using the Z-axis movable stage 112 (at this time, the Y axis and the X axis are adjusted in advance so as not to be moved). At the same time as turning on the switch of a vibrator 109, a magnet plate 117 (such that the magnet is arranged at the center of one well out of four wells in 96- wells) is pushed up toward the 96-well plate 115 by a Z-axis movable stage 118. Thus, the diffused beads can be collected. At this time, a damper 110 is a rubber block in which the vibration of the vibrator is not transmitted to the entire device. Further, the vibrator is connected to a Y-axis movable stage 111 for arranging the vibrator at an appropriate position. 116 denotes a block that fixes the plate so as not to move upward, and a vibrator (automation of a buffer dispensing mechanism is required for automation) may be housed for automation of the step of washing beads. 106 denotes a controller that is configured to control the stages 102 and 103, the vibrator 109, and the like in accordance with control from the PC.

After waiting for a time (about 10 seconds) until the beads are collected, the container for single cell analysis and the vibration bar are lifted from the plate, and the treatment is completed.

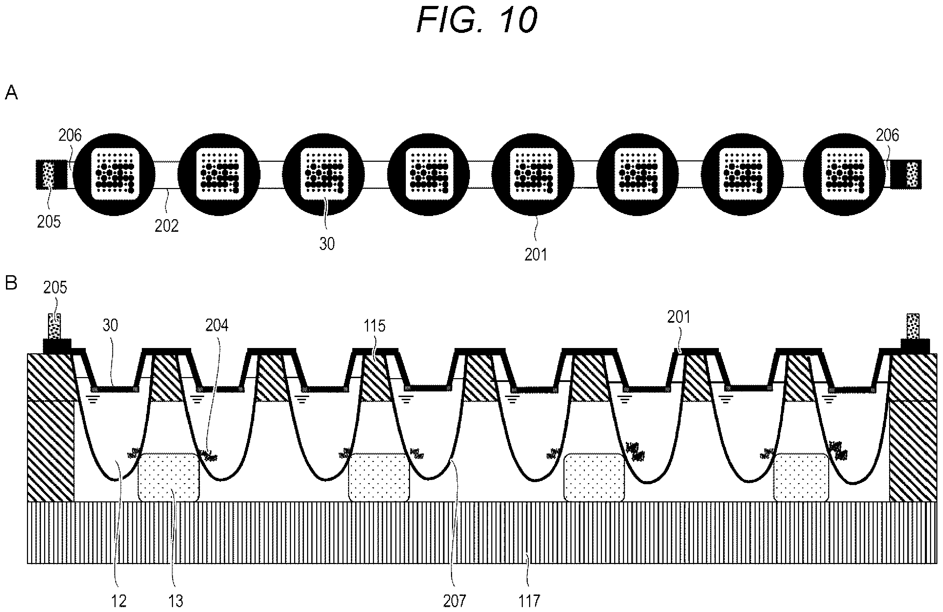

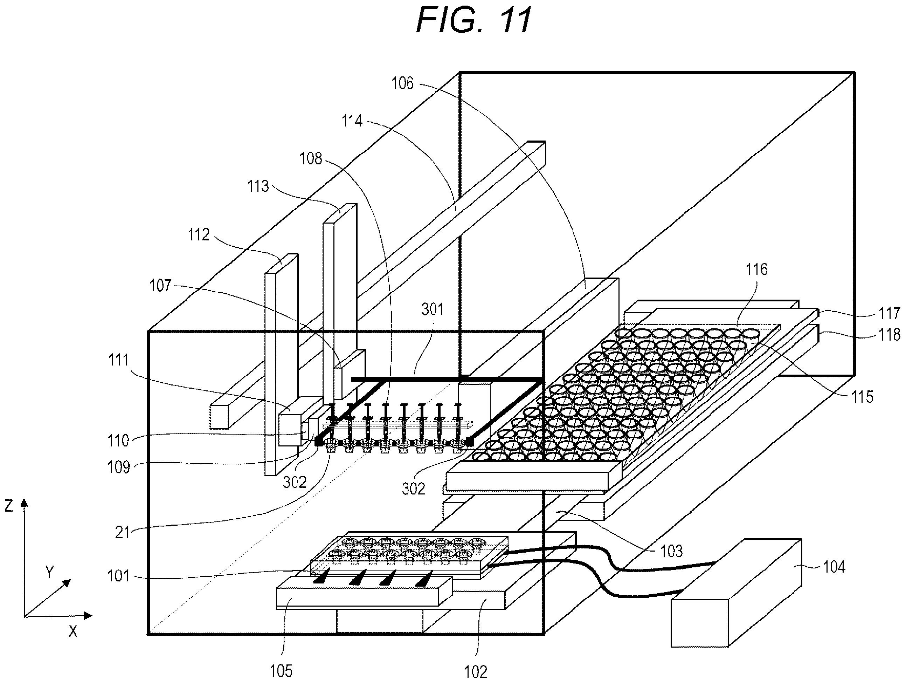

There are other methods of lifting the container for single cell analysis and immersing (at least) a part of the container in the solution of the well in the 96-well plate 115 for collecting beads. Particularly, an effective method is a method of connecting a plurality of containers, collectively lifting the connected containers for single cell analysis 201, and collectively immersing the containers. The structure of the containers for realizing this method is shown in FIG. 10, and a configuration example of the device is shown in FIG. 11. FIG. 10A illustrates a top view of only the connected containers 201 from the upper surface of the chip 30, and FIG. 10B illustrates a cross-sectional view from the side in a state where the connected containers 201 are inserted into the 96-well plate 115. In this example, the number of connected containers is 8, and the connected containers are arranged at intervals of 9 mm such that the connected containers can be inserted into the 96-well plate. A stainless steel magnet fixing block 205 is provided at both ends of the plate such that the connected containers 201 can be lifted using a magnet and inserted into wells of the plate. At this time, a connection portion 202 for fixing the interval between the containers is also made of the same resin as the resin container. Of course, instead of the 96- well plate, a 384 plate (4.5 mm interval), an 8-row tube (9 mm interval), or the like may be used. As a fixing method of lifting the connected containers, in addition to the magnet, recesses and protrusions such as wells (pick-up wells 206) at both ends of the connected containers are used, and the connected containers may be hooked on the projections on the pawls and lifted. Alternatively, the connected containers may be fixed and moved by using a robot hand (arm) capable of electrically "gripping" objects.

When collecting beads using the device, the connected containers 201 are inserted into wells 207 of the plate 15, and the volume of the buffer is adjusted such that the bead suspension and collection buffer (solution) comes into contact with the single cell analysis chip 30 (about 150 to 200 µL). A magnet (neodymium magnet) 302 at the ends of the arm is fixed to the magnet fixing block 205 by magnetic force using a magnet fixing arm 301 shown in the configuration of the device in FIG. 11, and the operation of lifting the connected containers and immersing them in the wells of the plate is performed. After the immersion of the connected containers 201, a vibrator rod with a disposable chip is brought into contact with the chip. Vibration is applied to the chip 30 using the vibrator 109, whereby the beads 204 in the buffer 12 are collected using the magnet 13 (FIG. 10B). The magnet is fixed on the magnet plate 117, and one magnet is allocated to four of the plate-like wells 207. After the collection of the beads, the connected containers were separated from the 96-well plate by lifting the arm 301. Further, the magnet plate 117 were relatively separated from the 96-well plate, and thus the beads could be resuspended in the solution.

In addition, the method of lifting the connected containers may be used for containers that are not connected.

Further, the structure of the connected containers may be applied to the structure of the device of Example 1 without an automated reactor.

All publications, patents, and patent applications cited herein are hereby incorporated by reference in their entirety.

### Reference Signs List

1 cell suspension
2 container for holding cell suspension
3 upper plate
4 aspiration flow path
5 lower plate
6 single cell analysis chip (VFACs: Vertical Flow Array Chips)
7 cell trapping portion
8 nucleic acid trapping portion (microreaction chamber)
9 porous membrane for holding beads
10 tweezers
11 resin tube for bead collection (PCR tube)
12 bead suspension and collection buffer (solution)
13 (neodymium) magnet
21 container for single cell analysis
22 intermediate plate for in-plane positioning of container for single cell analysis
23 upper plate for pressing container for single cell analysis
24 positioning pin
25 sealing rubber for single cell analysis chip
26 rod fixing projection
27 rod for lifting container for single cell analysis
28 vibration bar
29 rod inserted state
30 single cell analysis chip (VFACs: Vertical Flow Array Chips)
31 cells (trapped on chip)
101 single cell analysis device
102 XZ-axes movable stage for single cell analysis device + plate-like heater (up to 50°C)
103 Y-axis movable stage for single cell analysis device
104 aspiration pump
105 blade for removing upper plate and blade holder
106 controller for stage, pump, vibrator, etc.
107 pickup arm for picking up container for single cell analysis (rod)
108 vibrator rod with disposable chip (vibration bar)
109 vibrator
110 damper (prevention of transmission of vibration to system)
111 Y-axis movable stage for vibrator rod
112 Z-axis movable stage for vibrator rod
113 Z-axis stage for lifting container
114 Y-axis stage for moving container
115 96-well plate (or 0.5 mL/1.5 mL tube array)
116 96-well plate fixing block (with vibrator)
117 magnet plate
118 Z-axis stage with Peltier Cooler for 96 plate
201 coupled container for single cell analysis
202 connection component between containers (made of resin)
204 beads
205 magnet fixing block
206 pick-up well
207 well
301 magnet fixing arm
302 magnet (neodymium magnet)

### Sequence Listing Free Text

SEQ ID NOs: 1 to 96: artificial (synthetic oligonucleotide)

## Claims

1. A container for single cell analysis comprising:
a reaction substrate comprising a cell trapping portion on which a cell is trapped, and one or a plurality of microreaction chambers arranged immediately below the cell trapping portion and filled with a solid phase; and
a cell holding portion having the reaction substrate as a bottom and configured to hold a solution containing cells.

2. The container according to claim 1, further comprising a fixture for fixing the container to a single cell analysis device.

3. The container according to claim 1, further comprising an auxiliary tool for removing the container from a single cell analysis device.

4. The container according to claim 1, wherein the solid phase is a bead.

5. The container according to claim 1, wherein the solid phase is a magnetic bead.

6. The container according to claim 1, wherein a single cell is trapped per the cell trapping portion.

7. The container according to claim 1, wherein the reaction substrate comprises a layer having an aspiration through-hole provided on a side opposite to the cell holding portion.

8. A single cell analysis method using a single cell analysis device, in the single cell analysis device to which the container according to claim 1 is fixed, the method comprising the steps of:
introducing a solution containing cells into the container;
trapping the cells in the cell trapping portion;
reacting nucleic acid derived from the trapped cells in the microreaction chamber;
removing the container from the single cell analysis device after the reaction; and
collecting the solid phase from the removed container.

9. The method according to claim 8, wherein the step of collecting the solid phase comprises immersing the container in another container containing a solution and vibrating the container.

10. The method according to claim 9, wherein the solid phase is a magnetic bead, and the method comprises collecting the solid phase in the another container by a magnet, with the vibration or after the vibration.

11. An automated single cell analysis device comprising:
a single cell analysis device configured to fix the container according to claim 1;
a mechanism that is configured to remove the container from the single cell analysis device and move the container to another container;
a mechanism that is configured to vibrate the container in the another container; and
a control device,
wherein the control device is configured to remove the container from the single cell analysis device after a reaction in the single cell analysis device, and to control a movement of the container to the another container and a vibration of the container in the another container.

12. The device according to claim 11, wherein the control device is configured to control a fixture fixing the container to the single cell analysis device to be removed.

13. The device according to claim 11, wherein the control device is configured to control the container to be pressed against the single cell analysis device to prevent air leakage when aspirating a reaction solution from the container.
